# EUROPEAN PATENT SPECIFICATION

(11) **EP 1 539 281 B1**
(45) Date of publication and mention of the grant of the patent: **30.05.2007**
(21) Application number: 03755805.3
(22) Date of filing: 10.09.2003
(51) Int. Cl.: A61M 5/32

(54) **METHOD AND APPARATUS FOR EPIDERMAL DELIVERY OF A SUBSTANCE**
VERFAHREN UND VORRICHTUNG ZUR EPIDERMISCHEN VERABREICHUNG EINER SUBSTANZ
PROCEDE ET APPAREIL DESTINES A ADMINISTRER UNE SUBSTANCE PAR VOIE EPIDERMIQUE

(30) Priority: 10.09.2002 US 409193 P
(43) Date of publication of application: 15.06.2005
(73) Proprietor: Becton Dickinson and Company, Franklin Lakes, New Jersey 07417-1880 (US)
(72) Inventor: HAIDER, M., Ishaq, Morrisville, NC 27560 (US); CLARKE, Richard, P., Raleigh, NC 27606 (US); FENTRESS, James, K., Morrisville, NC 27560 (US); MIKSZTA, John, A., Durham, NC 27705 (US); MARTIN, Frank, E., Durham, NC 27704 (US)
(74) Representative: Weber, Thomas
(86) International application number: PCT/US2003/028273
(87) International publication number: WO 2004/024219

(56) References cited:
- DE-A- 19 512 607
- DE-A- 19 717 253
- DE-C- 446 818
- FR-A- 2 749 172
- US-B1- 6 200 291
- US-B1- 6 261 272

## Description

### Field of the Invention

The present invention relates generally to a needle and an apparatus for the transdermal delivery of a substance, and more particularly to a side-ported needle for the intradermal or epidermal delivery of fluids.

### Related Art

The importance of efficiently and safely administering pharmaceutical substances such as diagnostic agents and drugs has long been recognized. The use of conventional needles has long provided one approach for delivering pharmaceutical substances to humans and animals by administration through the skin. Considerable effort has been made to achieve reproducible and efficacious delivery through the skin while improving the ease of injection and reducing patient apprehension and/or pain associated with conventional needles. Furthermore, certain delivery systems eliminate needles entirely, and rely upon chemical mediators or external driving forces such as iontophoretic currents or electroporation or thermal poration or sonophoresis to breach the stratum corneum, the outermost layer of the skin, and deliver substances through the surface of the skin. However, such delivery systems do not reproducibly breach the skin barriers or deliver the pharmaceutical substance to a given depth below the surface of the skin and consequently, clinical results can be variable. Thus, mechanical breach of the stratum corneum, such as with needles, is believed to provide the most reproducible method of administration of substances through the surface of the skin, and to provide control and reliability in placement of administered substances.

Approaches fer delivering substances beneath the surface of the skin have almost exclusively involved transdermal administration, ie. delivery of substances through the skin to a site beneath the skin. Transdermal delivery includes subcutaneous, intramuscular or intravenous routes of administration of which, intramuscular (IM) and subcutaneous (SC) injections have been the most commonly used.

Anatomically, the outer surface of the body is made up of two major tissue layers, an outer epidermis and an underlying dermis, which together constitute the skin (for review, see Physiology, Biochemistry, and Molecular Biology of the Skin, Second Edition, L.A. Goldsmith, Ed., Oxford University Press, New York, 1991). The epidermis is subdivided into five layers or strata of a total thickness of between 75 and 150 µm. Beneath the epidermis lies the dermis, which contains two layers, an outermost portion referred to as the papillary dermis and a deeper layer referred to as the reticular dermis. The papillary dermis contains vast microcirculatory blood and lymphatic plexuses. In contrast, the reticular dermis is relatively acellular and avascular and made up of dense collagenous and elastic connective tissue. Beneath the epidermis and dermis is the subcutaneous tissue, also referred to as the hypodermis, which is composed of connective tissue and fatty tissue. Muscle tissue lies beneath the subcutaneous tissue.

As noted above, both the subcutaneous tissue and muscle tissue have been commonly used as sites for administration of pharmaceutical substances. The dermis, however, has rarely been targeted as a site for administration of substances, and this may be due, at least in part, to the difficulty of precise needle placement into the intradermal space. Furthermore, even though the dermis, in particular the papillary dermis, has been known to have a high degree of vascularity, it has not heretofore been appreciated that one could take advantage of this high degree of vascularity to obtain an improved absorption profile for administered substances compared to subcutaneous administration. This is because small drug molecules are typically rapidly absorbed after administration into the subcutaneous tissue which has been far more easily and predictably targeted than the dermis has been. On the other hand, large molecules such as proteins are typically not well absorbed through the capillary epithelium regardless of the degree of vascularity so that one would not have expected to achieve a significant absorption advantage over subcutaneous administration by the more difficult to achieve intradermal administration even for large molecules.

One approach to administration beneath the surface to the skin and into the region of the intradermal space has been routinely used in the Mantoux tuberculin test. In this procedure, a purified protein derivative is injected at a shallow angle to the skin surface using a 27 or 30 gauge needle (Flynn et al, Chest 106: 1463-5, 1994). A degree of uncertainty in placement of the injection can, however, result in some false negative test results. Moreover, the test has involved a localized injection to elicit a response at the site of injection and the Mantoux approach has not led to the use of intradermal injection for systemic administration of substances.

Some groups have reported on systemic administration by what has been characterized as "intradermal" injection. In one such report, a comparison study of subcutaneous and what was described as "intradermal" injection was performed (Autret et al, Therapie 46:5-8, 1991). The pharmaceutical substance tested was calcitonin, a protein of a molecular weight of about 3600. Although it was stated that the drug was injected intradermally, the injections used a 4 mm needle pushed up to the base at an angle of 60°. This would have resulted in placement of the injectate at a depth of about 3.5 mm and into the lower portion of the reticular dermis or into the subcutaneous tissue rather than into the vascularized papillary dermis. If, in fact, this group injected into the lower portion of the reticular dermis rather than into the subcutaneous tissue, it would be expected that the substance would either be slowly absorbed in the relatively less vascular reticular dermis or diffuse into the subcutaneous region to result in what would be functionally the same as subcutaneous administration and absorption. Such actual or functional subcutaneous administration would explain the reported lack of difference between subcutaneous and what was characterized as intradermal administration, in the times at which maximum plasma concentration was reached, the concentrations at each assay time and the areas under the curves.

Similarly, Bressolle et al. administered sodium ceftazidime in what was characterized as "intradermal" injection using a 4 mm needle (Bressolle et al. J. Pharm. Sci. 82:1175-1178, 1993). This would have resulted in injection to a depth of 4 mm below the skin surface to produce actual or functional subcutaneous injection, although good subcutaneous absorption would have been anticipated in this instance because sodium ceftazidime is hydrophilic and of relatively low molecular weight.

Another group reported on what was described as an intradermal drug delivery device (U.S. Patent No. 5,997,501). Injection was indicated to be at a slow rate and the injection site was intended to be in some region below the epidermis, i.e., the interface between the epidermis and the dermis or the interior of the dermis or subcutaneous tissue.

The standard needle used for subcutaneous or transdermal injections is in the form of a hollow shaft provided with a sharpened open end. This type of needle has been found to be unreliable in ensuring accurate delivery rates, in particular when used in conjunction with an infusion pump or some or other delivery device which depends for delivery on the magnitude of pressure applied to the liquid being delivered.

The lack of accuracy with standard needles is thought to be due to the build up of pressure at the delivery point which opposes the driving pressure applied to the liquid. For example, in experiments in which a saline solution is infused intradermally, the fluid path to the needle is initially blocked and a high pressure (peak pressure) is required to remove the blockage prior to settling down at a lower steady state delivery pressure.

Others have tried to combat these problems by designing needles which are intended to avoid the problem of pressure build up arising from the orifice becoming plugged by tissue upon entry of the needle into the skin. For example, U.S. Patent Nos. 6,261,272 and 6,346,095 to Gross describe a needle having one or more apertures located on the side of the needle shaft, located in the vicinity of the sharpened tip of the needle. The needles described in Gross are designed for subcutaneous and intramuscular injection of a substance. As such, the needles of the Gross patents project 5 mm into the skin and deliver their payload at about that depth, which is far below the epidermis.

In order for many types of drugs or vaccines to be effective, it is important that the drug or vaccine be delivered to the epidermis. For example, "Cutaneous Vaccination: The Skin as an Immunologically Active Tissue and The Challenge of Antigen Delivery", S. Babiuk et al, Journal of Controlled Release, pps. 199-214, 2000 describes the importance of epidermal delivery of vaccine antigens. To be effective, the vaccine antigens should be delivered to the epidermis where the antigen presenting cells are present

There has been an increased interest in microneedle injection for the transdermal delivery of various drugs. Microneedle devices may include one or plurality of microneedles with a length of a few hundred microns to a few millimeters. Microneedle drug delivery devices are able to penetrate the stratum corneum of the skin with less irritation.

Thus, there is a need for a drug delivery method and device for delivering drugs or vaccines to the epidermis.
It is an object of the invention to provide a needle and an infusion apparatus causing improved absorption profile for substances administered to the skin of a patient.

### Summary of the Invention

This object is solved with the features of claim 1 and 11 respectively.

An apparatus for epidermal and/or intradermal delivery of a substance is provided. A needle having at least one side port can be used to penetrate the skin of a subject. The needle may be of any size. A substance can be delivered through the side port and into the skin. The side port can be of any size or shape and be arranged at any location on the needle.

The needle comprises a shaft having a wall defining a longitudinally extending bore, a first end that is open to receive a substance in the bore, a second end adapted to penetrate skin of a subject, and a penetration length of less than about 4.5 mm; and at least one side port extending through the wall and communicating with the bore.

The infusion apparatus comprises a housing including a reservoir for containing a supply of liquid medication and for delivering the liquid medication under pressure; a delivery cannula carried by the housing, the delivery cannula including a side port communicating with an interior of the cannula, the side port being disposed about .025 mm - to about 2,5 mm below a surface of the skin when the needle is inserted into the skin; and a flow channel for conducting the liquid medication from the reservoir to the delivery cannula.

In an exemplary embodiment, the side port is arranged at depth below the skin to deliver the substance to the epidermis.

In another embodiment, the side port can be arranged to deliver the substance into the intradermal space.

In a further embodiment, the needle is provided with multiple side ports. These side ports may be arranged at different depths below the surface of the skin.

In another embodiment, the needle is provided with an end port in addition to at least one side port.

Further features and advantages of the invention, as well as the structure and operation of various embodiments of the invention, are described in detail below with reference to the accompanying drawings.

### Brief Description of the Drawings

The foregoing and other features and advantages of the invention will be apparent from the following, more particular description of a preferred embodiment of the invention, as illustrated in the accompanying drawings wherein like reference numbers generally indicate identical, functionally similar, and/or structurally similar elements. The left most digits in the corresponding reference number indicate the drawing in which an element first appears.

FIGS. 1a - 1b depict exemplary embodiments of a needle according to an embodiment of the present invention;

FIG. 2 depicts an exemplary embodiment of an infusion device according to another embodiment of the present invention; and

FIG. 3 depicts an exemplary embodiment a needle according to another embodiment of the present invention.

### Detailed Description of an Exemplary Embodiment of the Present Invention

A preferred embodiment of the invention is discussed in detail below. While specific exemplary embodiments are discussed, it should be understood that this is done for illustration purposes only. A person skilled in the relevant art will recognize that other components and configurations can be used without parting from the invention.

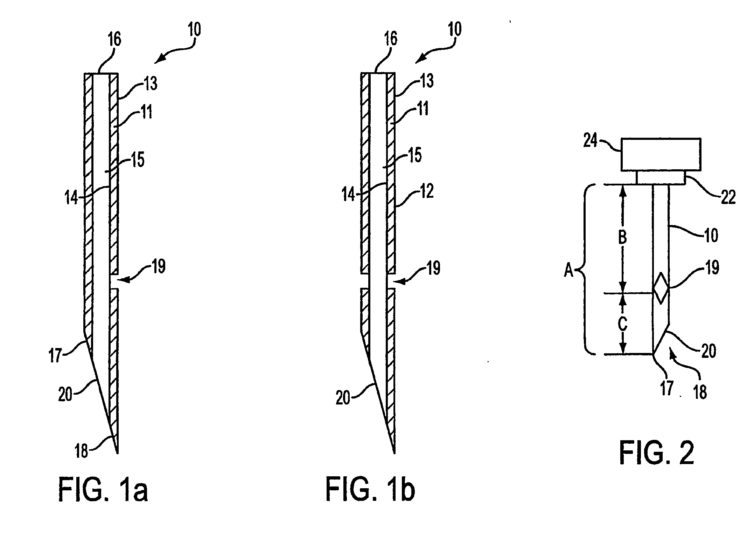

In FIG. 1 there is indicated, generally at 10, a needle according to an embodiment of the invention. The needle 10 comprises a shaft 11 including a wall 12. The wall 12 includes an external surface 13, an internal surface 14 and defines a longitudinally extending internal bore 15.

The bore 15 is open at first end 16 to receive a supply of drugs or other substance for delivery, for example by connection to a drug pump, infusion device or a syringe. A second end 17 of the needle 10 is adapted to penetrate the skin of a subject. The end 17 of the needle 10 is sharpened with a beveled tip 18. The beveled tip 18 may have any shape, for example, a tapered shape. End 17 of the needle 10 may either be open or closed depending on the needle's application. A terminal orifice or end port 20 at end 17 of the shaft 11 may further facilitate communication between the bore 15 and the exterior of the needle 10.

The shaft 11 is provided with at least one aperture (side port) 19. The side port 19 may be spaced from the end 16 and have any shape or size. The side port 19 permits communication between the internal bore 15 and the exterior of the needle 10. Thus, the side port 19 should extend through wall between the exterior surface 13 and interior surface 14 and open into bore 15. The side port 19 can be arranged at any location on the shaft 11. For example the side port 19 may be arranged on a side of the shaft 11 opposite from the bevel 18, as shown in figure 1. In another embodiment of the invention shown in figure 1b, two side ports 19 are provided on the shaft 11. The side ports 19 are arranged on opposite sides of the shaft. Of course, many other arrangements, locations, and numbers of side ports 19 are possible within the scope of the invention.

Providing needle 10 with one or more side port(s) 19 permits greater flexibility in the needle design and manufacturing process than prior needles. For example, when delivering a drug or vaccine to the epidermis, a needle having a penetration length of less than .5 mm is typically used, as the epidermis is extremely thin. Due to the short penetration depth of the needle, significant problems regarding drug leakage can occur. Shorter needle lengths (i.e., <1.5 mm) may have inherent leakage problems because of the improper needle seating into the tissue. Additionally, the substance tends to effuse out of the skin due to backpressure exerted by the skin itself and to pressure built up from accumulating fluid from the injection or infusion. The problem is minimized as the length of the needle is increased. One way to mitigate the problem is to use longer needles with the end-port blocked but the side port located in the intradermal region, or alternatively, the epidermal region. For example, such needles were tested in animal trials using 2 to 3 mm long needles with the end port blocked. The side port of the same needles were 1.0 to 1.5 mm below the surface of the skin. These needles did not show any signs of leakage because of improvement in their seating. Additionally, it is difficult to manufacture a needle that only penetrates the epidermis .5 mm. Needle handling is an important part of the device manufacturing process. Longer needle lengths are easier to handle and therefore easier to manufacture as compared to shorter length needles.

As used herein, intradermal is intended to mean administration of a substance into the dermis in such a manner that the substance readily reaches the richly vascularized papillary dermis and is rapidly absorbed into the blood capillaries and/or lymphatic vessels to become systemically bioavailable. Such can result from placement of the substance in the upper region of the dermis, i.e. the papillary dermis or in the upper portion of the relatively less vascular reticular dermis such that the substance readily diffuses into the papillary dermis. It is believed that placement of a substance predominately at a depth of at least about 0.3 mm, more preferably, at least about 0.4 mm and most preferably at least about 0.5 mm up to a depth of no more than about 2.5 mm, more preferably, no more than about 2.0 mm and most preferably no more than about 1.7 mm will result in rapid absorption of macromolecular and/or hydrophobic substances.

In order to deliver fluids intradermally at a desired depth below the skin surface, for example about 1 mm below the skin surface, and yet use longer needle lengths, for example longer than 1 mm, one approach is to incorporate the side port(s) 19 at the desired depth below a hub on a longer needle. A single side port or multiple side ports 19 can be arranged at any location along the shaft 11 to deliver a substance at various depths shallower than the length of the needle. In exemplary embodiments of the invention, one or more of the side ports are arranged on the shaft to deliver a substance epidermally and/or intradermally and/or subcutaneously and/or intramuscularly or any combination thereof.

The needle 10 should have a penetration length of about 4.5 mm or less. Penetration length is defined as the extent to which the needle penetrates below the surface of the skin. The overall length of the needle may be different from the penetration length. For example, the needle may be secured to a housing of a syringe, an infuser or insulin pen. A portion of the needle may extend into the housing to secure the needle in the housing, making the overall length of the needle greater than 4.5 mm, but the needle may still only penetrate the skin less than 4.5 mm.

Figure 2 illustrates the definition of penetration length. The needle 10 is attached to a delivery device having a hub 22. A portion 24 of the needle extends inside the hub 22. Beveled tip 18 is pressed against the skin of a subject and the needle is inserted into the skin. The needle 10 is inserted until hub 22 comes into contact with the surface of the skin. The needle 10 shown in Figure 2 thus has a penetration length A, from end 17 to hub 22. The portion 24 of the needle 10 inside the hub 22 does not penetrate the skin and does not constitute part of the penetration length A. In an exemplary embodiment, the needle 10 has a penetration length of about 4.5 mm, and preferably about 3 mm, or less.

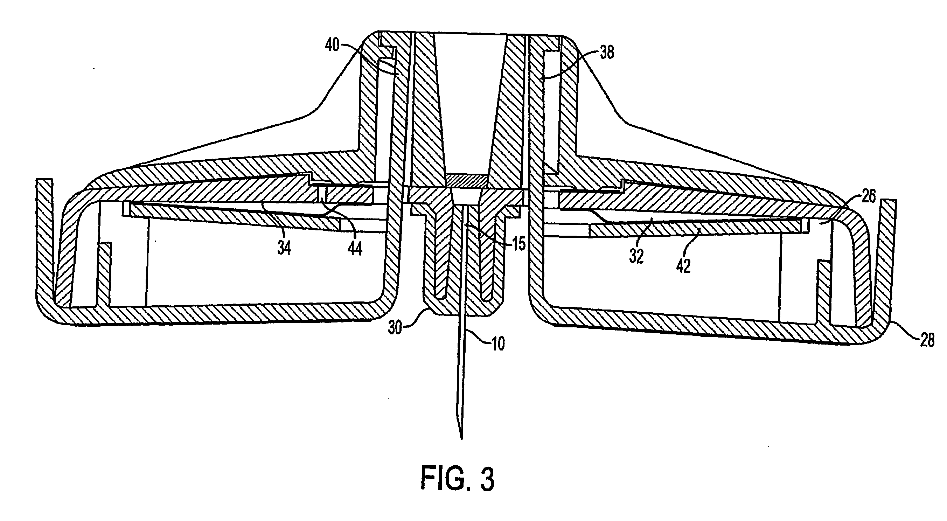

Needles according to embodiments of the invention may be used in conjunction with infusers, insulin pens, and other drug delivery devices. An exemplary embodiment of an infusion device is shown in figure 3. The two major components of the infusion device are a top cover 26 and a bottom cover 28, which comes in contact with the skin of the user. The needle 10 for delivering a substance is retained into a hub 30, which is attached to the top cover 26. A bladder membrane 32 is provided on an inside surface 34 of the top cover 26. The substance to be delivered may be contained between the bladder membrane 32 and the inside of the top cover 26. The assembly is held together by two legs, 38 and 40 on the bottom cover 28, which reach up through holes in the top cover 26. A spring 42 may be retained to the bottom cover 2.

As the top cover 26 collapses into the bottom cover 28, the spring 42 is forced into contact with the bladder 32 containing the substance. This spring force causes the spring 42 to deflect downward and imparts a precise pressure upon the substance in the bladder 32.

Once the bladder 32 is filled with a substance and pressurized by the contact with spring 42, the only exit path for the substance is through the small passage 44 in the top cover 26. The substance flows through passage 44 and a channel to the end 16 of the needle 10 so that it is free to flow into the skin of the user.

The infusion device and needle are used for delivery of a substance into the skin. According to an exemplary embodiment of the invention, the needle is adapted to penetrate below a selected layer of skin to which delivery of a substance is desired, yet deliver the substance to the selected layer. For example, needle 10 can be adapted to penetrate into the intradermal layer and deliver a substance to the epidermal layer. A needle 10 with side port 19 and a closed end 17 can be used for this epidermal delivery. The needle 10 is inserted into the skin of a subject using the above-described infusion device. Typically, the needle 10 is inserted into the skin at an angle that is substantially perpendicular to the surface of the skin, between 80-90 degrees. Preferably, the insertion angle should be greater than 45 degrees. When inserted, the needle should only penetrate into the intradermal space, and preferably does not penetrate into the subcutaneous layer of the skin. For example, the needle 10 may have a penetration length of about 1 mm to about 3 mm.

The side port 19 is adapted to deliver the substance to the epidermis of the subj ect. The side port 19 can be arranged on the shaft 11 of the needle 10 such that the side port 19 is about .025 mm to about 3 mm below the surface of the skin when the needle 10 is inserted into the skin of a subject. When delivering certain substances, such as vaccines, side port 19 should be about .025-1.5 mm below the surface of the skin when the needle 10 is inserted into the skin of a subject. In some instances, the needle penetrates below the selected layer of skin to which delivery is desired, but can deliver the substance to the selected layer.

It has been found that with the present invention the substance can be delivered at a substantially constant pressure and constant delivery rate, without the usual plugging or increase in delivery pressure necessary with conventional, non-side ported needles. The present invention also showed a significant reduction in the amount of pressure required to initiate and to continue at a constant pressure intra-dermal infusion. This allows for the creation of infusion devices that utilize less force, and thus, less pressure. This also allow the creation of smaller devices, and devices that do not have to be engineered to sustain high forces. Prior art devices, particularly constant pressure devices utilizing non side-ported needles, have to utilize a much higher pressure to insure that infusion takes place 100% of the time. One possible theory to explain why side ported needles overcome the need for similarly high infusion pressures is that non-ported needles whose flow pathway is parallel to the insertion path of the needle may experience a localized occlusion or sealing at the needle tip. The addition of a side port allows for a flow path that is perpendicular to the insertion path of the needle, and thus can overcome any localized effects related to the needle tip. In addition, the flow path generated by a side-ported needle may be better able to perfuse laminar skin physiology. Providing needles with side ports can greatly reduce the pressure requirements of shorter needles.

In order to deliver the substance, the substance is introduced to the bore 15 of the needle. The substance, which may be a drug or vaccine, is then provided from the bore 15, through the side port 19 into an area of the skin contiguous with the side port 19. Delivery of the substance occurs through the side port to the desired layer of skin, such as the epidermis. The delivery of the substance from the side port 19 is usually in a transverse direction, that is, perpendicular to the insertion path of the needle 10. In most cases the transverse path is substantially parallel to the surface of the skin.

Accordingly, the needle penetrates into the intradermal layer, and delivers a payload to the epidermis. By providing a needle 10 with a side port 19 and a closed end 17, the needle 10 may have a longer overall length and the attendant manufacturing and delivery advantages described above, and still be able to perform epidermal delivery with no leakage and reduced pressure compared to prior devices.

As just discussed, a needle having a closed end 17 and side port 19 is extremely useful to deliver drugs to the epidermis. Some drugs and vaccines, such as DNA and polysaccharide polymer vaccines, have better efficacy when they are delivered as shallow as possible into the epidermis. A needle 10 having a side port 19 arranged about .5 mm or less from the surface of the skin when the needle 10 is inserted into a subject can be used for epidermal delivery of DNA and polysaccharide polymer vaccines. The side port 19 may be arranged on the needle 10 such that the vaccine is delivered to the epidermis from under the epidermis.

In another exemplary embodiment of the invention, the needle 10 and side port 19 are adapted for intradermal delivery of a substance. In this embodiment, 30 gauge and 31 gauge needles are used for intradermal delivery. The needles are intended to effect the delivery at a depth of about 1 mm under the surface of the skin. The penetration length of the needle may range from about 1.5 mm to about 3 mm. Referring again to figure 2, an example of a needle adapted for intradermal delivery is described. The needle 10 has a penetration length A, which here is about 1.5 mm. The side port 19 is arranged a distance B from the hub 22 for intradermal delivery. Here, distance B is about 1 mm. The side port 19 is arranged a distance C from the second end 17 of the needle 10. Here, distance C is about .5 mm. Needles of these types can be used in insulin pens. Also, using larger diameter cannula for intradermal delivery allows for lower delivery pressures and ease of manufacturing.

Turning now to another embodiment of the invention, needle 10 is provided with an opening 20 at its end 17. The opening 20 can be used in addition to one or more side ports 19 for the delivery of drugs or vaccines. A substance can be delivered simultaneously through the side port(s) 19 for absorption into one or more layers of skin and through the end port 20 for absorption into another layer of skin. The first and second layers of skin may be different from each other. This allows bi-phasic delivery of drugs; that is, the drug can be deliver at two different depths. Furthermore, when multiple side port are provided triphasic deliver to the epidermal, intradermal, and subcutaneous space can be achieved.

For example, a needle may have a penetration depth of about 3 mm or less to penetrate into the intradermal layer. The needle can be provided with a side port 19 that is adapted to deliver the substance to the epidermis, for example a side port arranged at a depth of less than .5 mm below the skin surface upon needle penetration. A drug delivered through the side port 19 at this depth has pharama-kinetic characteristics very similar to a drug delivered intravenously. The end port 20 is arranged at the end 17 of the needle 10, in an intradermal region about 3 mm below the surface of the skin upon full penetration by the needle. A drug delivered through opening 20 enters the dermis and requires a longer period of time for absorption. Accordingly, a drug can be delivered simultaneously to different layers of the skin such as the epidermis and intradermal layer and absorption of the drug can be controlled.

Additionally, providing one or more side ports and/or an open-end port results in an increase in the volume of fluid that can be delivered. It has been determined in various studies that the volume limitation for intradermal bolus delivery is around 200 to 250 microliters per needle site. This is assumed to be related to the biological limitation of the intradermal tissue at the point of fluid administration. A method to increase the fluid volume in the intradermal space is to introduce the fluid at two tissue layers and both located in the intradermal region. For example, 1.5 mm long needles (total length) with a side port located at 0.5 to 1.5 mm below the skin surface can deliver fluids into two distinct layers both located in the intradermal space. Therefore, introducing fluids via two ports into the intradermal space can have a similar effect as if the fluid was introduced by two needles from, the same array. The net result is to increase the fluid volume administered to the intradermal space.

The back pressure at the end port (1.5 mm below the skin surface) is actually lower than the region closer to the skin surface where the side port is located. To enable fluid delivery via the side port when the needle 10 is provided with an open end 17, it is important that the pressure at the needle end 17 is higher than the pressure at the side port region. This condition can be caused by (a) tissue compaction at the end port 20 that can cause flow blockage, and, (b) a larger pressure gradient between the needle entrance and the needle tip as compared to the gradient between the needle entrance and the sideport. Alternatively, a needle may be fabricated with a partial blockage at the tip. Such needles exhibit higher pressures at the needle end 17 because of a diameter reduction near needle end 17.

In an exemplary embodiment, a 34 exterior gauge (or smaller) needle is provided with a single or multiple side ports. The needle has a penetration length of less than 3 mm and a beveled tip. The beveled tip has a 28° bevel angle. : The side port is provided in the vicinity of the needle tip, preferably on the opposite side of the bevel opening. The needle is used in an infusion device. By using such a needle with side ports, the operating pressure of the infusion device can be reduced to less than 34.5 kPa (5 psi). Needles according to this embodiment of the invention can be very useful for the design of small and portable infusion pumps, where the size is consequential to the function of the device.

Experimental tests performed illustrated the ability of embodiments of the invention to perform bi-phasic shallow intradermal delivery through a side port and deeper intradermal delivery through an end port. These tests were performed to deliver 60 microliters of fluid using a 34 gauge needle having a penetration length of 1.5 mm with a side port located .5 mm below the skin surface and to deliver 100 microliters of fluid using a 31 gauge needle having a penetration length of 2.0 mm and a side port located 1.0 mm below the skin surface.

Experimental tests performed illustrated the ability of embodiments of the invention to perform shallow epidermal delivery through a side port. These tests were performed to deliver 70 microliters of fluid using a 34 gauge needle having a penetration length of 1.4 mm with a side port located .4 mm below the skin surface and to deliver 60 microliters of fluid using a 34 gauge needle having a penetration length of 1.5 mm and a side port located .5 mm below the skin surface. These tests resulted in fluid delivery localized completely in the shallow intradermal tissue with spreading within and just under the epidermis.

Moreover, by adding side ports to the needle, the fluid delivery capacity of the needle can be enhanced. This is very important for the application of complex fluids both in conventional large diameter needles and small needles suitable for intradermal delivery. Complex fluids can include (a) highly viscous biological fluids such as proteins, DNA, etc., and (b) non-homogeneous two-phase solutions. An example of the second category is microspheres based suspension drugs used for intramuscular injection. Such drug formulations, microspheres suspended in liquid diluent, normally require larger gauge needles (i.e., 18 gauge) to remedy clogging problems. In statistically designed experiments using animal tissues, it has been determined that side-ported needles eliminate the clogging problems. As a result smaller gauge needles i.e., 21 gauge instead of the 18 gauge may be used for the application to reduce pain. Additionally, in some cases, the side port can have a slightly greater penetration depth and the epidermis can be approached from its underside. In most instances, upon full needle penetration, the side port is closer to the surface of the skin that to the inserted end of the needle.

While various embodiments of the present invention have been described above, it should be understood that they have been presented by way of example only, and not limitation. Thus, the breadth and scope of the present invention should not be limited by any of the above-described exemplary embodiments, but should instead be defined only in accordance with the following claims.

## Claims

1. A needle (10), for delivery of a substance to the skin of a patient, having a skin contacting surface (22) and comprising:
a shaft (11) having a wall (12) defining a longitudinally extending bore (15),
a first end (16) that is open to receive the substance in the bore (15), and
a second end (17) adapted to penetrate skin of a subject,
wherein the second end (17) comprises a sharpened tip (18) and wherein the tip (18) is bevelled,
the shaft (11) having a penetration length (A) extending from the skin engaging surface (22) to a distal tip of the second end (17) of less than 4.5 mm, and
the shaft having at least one side port (19) extending through the wall (12) and communicating with the bore (15),
**characterized by**
the side port (19) being arranged 0.025 mm to 2.5 mm from the skin contacting surface (22), wherein, when the needle (10) is fully penetrated into the skin, the skin contacting surface (22) contacts the skin, and the substance exits the side port (19) under pressure directly into the skin.

2. The needle (10) of claim 1, wherein the side port (19) is arranged on a side of the shaft (11) opposite of the bevel of the tip (18).

3. The needle (10) of claim 1 or 2, wherein the second end (17) includes an end port (20) communicating with the bore.

4. The needle (10) of one of claims 1 - 3, wherein the side port (19) and the end port (20) are adapted for biphasic delivery of a substance.

5. The needle (10) of one of claims 1-4, wherein the side port (19) is arranged on the shaft (11) for epidermal delivery of a substance.

6. The needle (10) of one of claims 1 - 5, wherein the side port (19) is arranged on the shaft (11) for intradermal delivery of a substance.

7. The needle (10) of one of claims 1 - 6, wherein the side port (19) is adapted to be 0.5 mm to 1.5 mm below a surface of the skin when the needle (10) is inserted into a subject.

8. The needle (10) of one of claims 1 - 7, wherein the wall (12) and the bore (15) correspond to the dimensions of a 30, 31 or 34 gauge needle.

9. The needle (10) of one of claims 1 -8, wherein the side port (19) has a diameter which is adapted for flow at pressures less than 34.5 kPa (5 psi).

10. The needle of one of claims 1-9, wherein 200 micro liters to 250 micro liters of said substance are delivered through said side port (19).

11. An infusion apparatus, comprising:
a housing including a reservoir for containing a supply of liquid medication and for delivering the liquid medication under pressure;
a delivery cannula (10) carried by and extending from the housing, the delivery cannula (10) including a side port (19) communicating with an interior of the cannula (10),
wherein the delivery cannula (10) further comprises a bevelled tip (18); and a flow channel for conducting the liquid medication from the reservoir to the delivery cannula (10),
**characterized by**
the side port (19) being arranged 0.025 mm to 2.5 mm below a surface of the skin when the needle is inserted into the skin of a subject.

12. The apparatus of claim 11, wherein the bevelled tip (18) includes an end port (20) communicating with the interior of the cannula (10).

13. The apparatus of claims 11 or 12, wherein the side port (19) is arranged on a side of the cannula (10) opposite the bevelled tip (18).

14. The apparatus of one of claims 11 - 13, further comprising at least two side ports (19).

15. The apparatus of one of claims 11 - 14, wherein the side port (19) is arranged on the delivery cannula (10) 200 microns from the housing.

16. The apparatus of one of claims 11 - 14, wherein the side port (19) is arranged on the delivery cannula (10) 0.025 to 1.5 mm from the housing.

17. The apparatus of one of claims 11-16, wherein the delivery cannula (10) is adapted to penetrate only an intradermal layer.

18. The apparatus of one of claims 11 - 16, wherein 200 micro liters to 250 micro liters of said substance are delivered through said side port (19).

19. The apparatus of one of claims 11 - 18, wherein the delivery cannula (10) is corresponding to the dimensions of a 30, 31 or 34 gauge cannula.

20. The apparatus of one of claims 11 - 19, wherein the side port (19) has a diameter which is adapted for flow at pressures less than 34,5 kPa (5 psi), when the cannula (10) is fully penetrated into the skin, and the substance exits the side port (19) under pressure directly into the skin

## Patentansprüche

1. Nadel (10) zum Zuführen einer Substanz zu der Haut eines Patienten, mit einer Hautkontaktierfläche (22), wobei die Nadel ferner aufweist:
einen Schaft (11) mit einer eine in Längsrichtung verlaufende Bohrung (15) begrenzenden Wand (12),
ein erstes Ende (16), das zum Aufnehmen der Substanz in der Bohrung (15) offen ist, und
ein zweites Ende (17) zum Eindringen in die Haut einer Person,
wobei das zweite Ende (17) eine spitzzulaufende Spitze (18) aufweist und wobei die Spitze (18) abgeschrägt ist,
wobei der Schaft (11) eine Eindringlänge (A) von der Hautangreiffläche (22) zu einer distalen Spitze des zweiten Endes (17) von weniger als 4,5 mm aufweist, und
wobei der Schaft mindestens einen Seitenport (19) aufweist, der durch die Wand (12) verläuft und mit der Bohrung (15) in Verbindung steht,
**dadurch gekennzeichnet, dass**
der Seitenport (19) 0,025 mm bis 2,5 mm von der Hautkontaktierfläche (22) entfernt angeordnet ist, wobei bei vollständig in die Haut eingedrungener Nadel (10) die Hautkontaktierfläche (22) die Haut kontaktiert und die Substanz unter Druck aus dem Seitenport (19) direkt in die Haut austritt.

2. Nadel (10) nach Anspruch 1, bei der der Seitenport (19) an einer der Abschrägung der Spitze (18) gegenüberliegenden Seite des Schafts (11) angeordnet ist.

3. Nadel (10) nach Anspruch 1 oder 2, bei der das zweite Ende (17) einen mit der Bohrung in Verbindung stehenden Endport (20) aufweist.

4. Nadel (10) nach einem der Ansprüche 1-3, bei der der Seitenport (19) und der Endport (20) für eine Zweiphasen-Zuführung einer Substanz vorgesehen sind.

5. Nadel (10) nach einem der Ansprüche 1-4, bei der der Seitenport (19) zur epidermalen Zuführung einer Substanz an dem Schaft (11) angeordnet ist.

6. Nadel (10) nach einem der Ansprüche 1-5, bei der der Seitenport (19) zur intradermalen Zuführung einer Substanz an dem Schaft (11) angeordnet ist.

7. Nadel (10) nach einem der Ansprüche 1-6, bei der sich der Seitenport (19) 0,5 mm bis 1,5 mm unter der Hautfläche befindet, wenn die Nadel (10) in ein Subjekt eingesetzt ist.

8. Nadel (10) nach einem der Ansprüche 1-7, bei der die Wand (12) und die Bohrung (15) den Abmessungen einer 30-, 31- oder 34-Gauge-Nadel entsprechen.

9. Nadel (10) nach einem der Ansprüche 1-8, bei der der Seitenport (19) einen Durchmesser aufweist, der für eine Strömung unter Drücken von weniger als 34,5 kPa (5 psi) vorgesehen ist.

10. Nadel nach einem der Ansprüche 1-9, bei der 200 Mikroliter bis 250 Mikroliter der Substanz durch den Seitenport (19) zugeführt werden.

11. Infusionsvorrichtung mit:
einem Gehäuse mit einem Reservoir zum Aufnehmen einer Menge eines flüssigen Medikaments und zum Zuführen des flüssigen Medikaments unter Druck;
einer von dem Gehäuse gehaltenen und von diesem abstehenden Zuführkanüle (10), die einen mit dem Inneren der Kanüle (10) in Verbindung stehenden Seitenport (19) aufweist,
wobei die Zuführkanüle (10) ferner eine abgeschrägte Spitze (18) aufweist;
und einem Strömungskanal zum Leiten des flüssigen Medikaments aus dem Reservoir zu der Zuführkanüle (10),
**dadurch gekennzeichnet, dass**
sich der Seitenport (19) 0,025 mm bis 2,5 mm unter der Hautfläche befindet, wenn die Nadel in die Haut einer Person eingesetzt ist.

12. Vorrichtung nach Anspruch 11, bei der die abgeschrägte Spitze (18) einen mit dem Inneren der Kanüle (10) in Verbindung stehenden Endport (20) aufweist.

13. Vorrichtung nach Anspruch 11 oder 12, bei der der Seitenport (19) auf einer der abgeschrägten Spitze (18) gegenüberliegenden Seite der Kanüle (10) angeordnet ist.

14. Vorrichtung nach einem der Ansprüche 11-13, ferner mit mindestens zwei Seitenports (19).

15. Vorrichtung nach einem der Ansprüche 11-14, bei der der Seitenport (19) 200 Mikrometer von dem Gehäuse entfernt an der Zuführkanüle (10) angeordnet ist.

16. Vorrichtung nach einem der Ansprüche 11-14, bei der der Seitenport (19) 0,025 bis 1,5 mm von dem Gehäuse entfernt an der Zuführkanüle (10) angeordnet ist.

17. Vorrichtung nach einem der Ansprüche 11-16, bei der die Zuführkanüle (10) zum Eindringen nur in die intradermale Schicht vorgesehen ist.

18. Vorrichtung nach einem der Ansprüche 11-16, bei der 200 Mikroliter bis 250 Mikroliter der Substanz durch den Port (19) zugeführt werden.

19. Vorrichtung nach einem der Ansprüche 11-18, bei der die Zuführkanüle (10) den Abmessungen einer 30-, 31- oder 34-Gauge-Kanüle entspricht.

20. Vorrichtung nach einem der Ansprüche 11-19, bei der der Seitenport (19) einen Durchmesser aufweist, der für eine Strömung bei Drücken von weniger als 34,5 kPa (5 psi) vorgesehen ist, wenn die Kanüle (10) vollständig in die Haut eingedrungen ist, und die Substanz unter Druck aus dem Port (19) direkt in die Haut austritt.

## Revendications

1. Aiguille (10) pour administrer une substance dans la peau d'un patient, ayant une surface au contact de la peau (22) et comprenant :
un axe (11) possédant une paroi (12) définissant un alésage se prolongeant longitudinalement (15),
une première extrémité (16) ouverte afin de recevoir une substance dans l'alésage (15), et
une seconde extrémité (17) adaptée pour pénétrer dans la peau d'un sujet,
la seconde extrémité (17) comprenant un embout pointu (18), lequel embout (18) étant biseauté,
l'axe (11) ayant une longueur de pénétration (A) se prolongeant à partir de la surface s'engageant dans la peau (22) jusqu'à l'embout distal de la seconde extrémité (17) inférieure à 4,5 mm, et
l'axe ayant au moins un orifice latéral (19) se prolongeant à travers la paroi (12) et communiquant avec l'alésage (15),
**caractérisée en ce que**
l'orifice latéral (19) est disposé à une distance de 0,025 mm à 2,5 mm de la surface au contact de la peau (22), où, lorsque l'aiguille (10) pénétrant entièrement dans la peau, la surface au contact de la peau (22) est en contact avec la peau et la substance sort par l'orifice latéral (19) sous pression directement dans la peau.

2. Aiguille (10) selon la revendication 1, dans laquelle l'orifice latéral (19) est disposé d'un côté de l'axe (11) opposé au biseau de l'embout (18).

3. Aiguille (10) selon la revendication 1 ou 2, dans laquelle la seconde extrémité (17) comprend un orifice d'extrémité (20) communiquant avec l'alésage.

4. Aiguille (10) selon l'une quelconque des revendications 1 à 3, dans laquelle l'orifice latéral (19) et l'orifice d'extrémité (20) sont adaptés pour une administration d'une substance biphasique.

5. Aiguille (10) selon l'une quelconque des revendications 1 à 4, dans laquelle l'orifice latéral (19) est disposé sur l'axe (11) de façon à administrer une substance par voie épidermique.

6. Aiguille (10) selon l'une quelconque des revendications 1 à 5, dans laquelle l'orifice latéral (19) est disposé sur l'axe (11) de façon à administrer une substance par voie intradermique.

7. Aiguille (10) selon l'une quelconque des revendications 1 à 6, dans laquelle l'orifice latéral (19) est adapté de façon à être de 0,5 mm à 1,5 mm en dessous d'une surface de la peau lorsque l'aiguille (10) est insérée dans la peau d'un sujet.

8. Aiguille (10) selon l'une quelconque des revendications 1 à 7, dans laquelle la paroi (12) et l'alésage (15) correspondent aux dimensions d'une aiguille de diamètre 30, 31 ou 34.

9. Aiguille (10) selon l'une quelconque des revendications 1 à 8, dans laquelle l'orifice latéral (19) a un diamètre adapté pour un écoulement à des pressions inférieures à 34,5 kPa (5 psi).

10. Aiguille selon l'une quelconque des revendications 1 à 9, dans laquelle 200 à 250 microlitres de ladite substance sont administrés à travers ledit orifice latéral (19).

11. Appareil d'infusion, comprenant :
un logement comprenant un réservoir destiné à contenir une alimentation en médicament liquide et à administrer le médicament liquide sous pression ;
une canule d'administration (10) supportée par et se prolongeant à partir du logement, la canule d'administration (10) comprenant un orifice latéral (19) communiquant avec l'intérieur de la canule (10),
la canule d'administration (10) comprenant en outre un embout biseauté (18) ; et un canal d'écoulement destiné à alimenter le médicament liquide provenant du réservoir dans la canule d'administration (10),
**caractérisé en ce que**
l'orifice latéral (19) est disposé de 0,025 mm à 2,5 mm en dessous d'une surface de la peau lorsque l'aiguille est insérée dans la peau d'un sujet.

12. Appareil selon la revendication 11, dans lequel l'embout biseauté (18) comprend un orifice d'extrémité (20) communiquant avec l'intérieur de la canule.

13. Appareil selon les revendications 11 ou 12, dans lequel l'orifice latéral (19) est disposé d'un côté de la canule (10) opposé à l'embout biseauté (18).

14. Appareil selon l'une quelconque des revendications 11 à 13, comprenant en outre au moins deux orifices latéraux (19).

15. Appareil selon l'une quelconque des revendications 11 à 14, dans lequel l'orifice latéral (19) est disposé sur la canule d'administration (10) à une distance de 200 microns du logement.

16. Appareil selon l'une quelconque des revendications 11 à 14, dans lequel l'orifice latéral (19) est disposé sur la canule d'administration (10) à une distance de 0,025 à 1,5 mm du logement.

17. Appareil selon l'une quelconque des revendications 11 à 16, dans lequel la canule d'administration (10) est adaptée de façon à pénétrer uniquement dans une couche intradermique.

18. Appareil selon l'une quelconque des revendications 11 à 16, dans lequel 200 à 250 microlitres de ladite substance sont administrés à travers ledit orifice latéral (19).

19. Appareil selon l'une quelconque des revendications 11 à 18, dans lequel la canule d'administration (10) correspond aux dimensions d'une aiguille de diamètre 30, 31 ou 34.

20. Appareil selon l'une quelconque des revendications 11 à 19, dans lequel l'orifice latéral (19) a un diamètre adapté pour un écoulement à des pressions inférieures à 34,5 kPa (5 psi) lorsque la canule (10) pénètre entièrement dans la peau, et la substance sort par l'orifice latéral (19) sous pression directement dans la peau.
